# EUROPEAN PATENT APPLICATION

(11) **EP 4 418 275 A1**
(43) Date of publication of application: **21.08.2024**
(21) Application number: 22880956.2
(22) Date of filing: 07.10.2022
(51) Int. Cl.: G16H 10/00, A61B 5/00

(54) **BODY COMPOSITION METER, SERVICE SYSTEM, MEASUREMENT VALUE TRANSMISSION METHOD, MEASUREMENT VALUE TRANSMISSION PROGRAM, AND COMPUTER-READABLE NON-TRANSITORY STORAGE MEDIUM**

(30) Priority: 12.10.2021 JP 2021167533
(71) Applicant: Tanita Corporation, Tokyo 174-8630 (JP)
(72) Inventor: KOSE, Mariko, Tokyo 174-8630 (JP)
(74) Representative: Eisenführ Speiser
(86) International application number: PCT/JP2022/037660
(87) International publication number: WO 2023/063261

(57) **Abstract**

A body composition meter (14) receives, from a terminal device (12) on which an application for using a measurement value of the body composition meter (14) to provide a service to a user functions, address information of a server (16) to which the measurement value is to be sent. The body composition meter (14) then registers the received address information for each user and sends the measurement value to the server (16) specified by the registered address information. The application on the terminal device (12) uses the measurement value stored in the server (16) to provide the service to the user.

## Description

### CROSS-REFERENCE TO RELATED APPLICATION

This application claims the benefit of Japanese Patent Application No. 2021-167533 filed on October 12, 2021 in Japan, the contents of which are incorporated herein by reference.

### TECHNICAL FIELD

The present disclosure relates to a body composition meter, a service system, a measurement value transmission method, and a measurement value transmission program.

### BACKGROUND ART

In recent years, systems are built up that send a user's measurement value obtained by a body composition meter via a network to a server and store it there. Such systems allow a user to use a web service that uses a measurement value obtained by a body composition meter.

For example, a healthcare apparatus according to Patent document 1 (Japanese Patent Laid-Open Application No. 2020-162649) comprises a plurality of measuring devices and a server connected to each other via a network, where measurement values of one or more biological indicators indicating a user's biological information are acquired and sent to the server.

### SUMMARY OF THE INVENTION

### Problems to be solved by the invention

In such a system as described in Patent document 1, the server to which a measurement value is to be sent is usually fixed, and a user can use a web service that is provided by the fixed destination server and uses the user's measurement value.

On a related note, some users may desire to use another web service using a measurement value, and such another web service using a measurement value may be provided by a server other than the fixed destination server. So-called inter-server coordination would be performed in order to achieve this, where the other server and the fixed destination server communicate with each other and the other server acquires the user's measurement value stored in the fixed destination server. While inter-server coordination includes file transfer, API (Application Programming Interface), and other methods, it requires time and cost for its preparation, arrangement, and the like.

Now, for example, if a body composition meter sends its measurement value directly to a server of a web service used by a user, it can be expected that the process to send the measurement value to the server of the web service will be facilitated and user convenience will be improved.

A purpose of the disclosure made in view of the above is to provide a body composition meter, a service system, a measurement value transmission method, and a measurement value transmission program that allow a body composition meter to send a measurement value directly to a server of a web service that a user uses.

### Means for solving the problems

A body composition meter of an aspect of the disclosure comprises: communication means for receiving, from a terminal device capable of communicating with the body composition meter, address information of a server to which a measurement value of the body composition meter is to be sent; registering means for registering the address information received by the communication means; and transmission control means for sending the measurement value to the server specified by the address information registered by the registering means.

In this configuration, the body composition meter receives, from the terminal device, address information of a server to which a measurement value is to be sent, registers the address information, and sends the measurement value directly to the server specified by the registered address information. The configuration therefore allows the body composition meter to send a measurement value directly to a server of a web service that a user uses.

In the body composition meter described above, the registering means may register the address information for each user. This configuration allows a measurement value to be sent to a different server for each user.

In the body composition meter described above, the registering means may register a condition for sending the measurement value to the server in association with the address information, and the transmission control means may send the measurement value that satisfies the condition to the server specified by the address information associated with the condition. This configuration allows a measurement value desired by a user to be sent to a server.

In the body composition meter described above, the condition may be based on the measurement value's magnitude.

In the body composition meter described above, the condition may be based on the measurement value's acquired date and time or day of week.

In the body composition meter described above, the registering means may register a server chosen by a user, as the server to which the measurement value is to be sent. This configuration allows a user to easily change a server to which a measurement value is to be sent.

A service system of an aspect of the disclosure comprises: a terminal device capable of communicating with a body composition meter; a body composition meter described above; and a server for holding a measurement value of the body composition meter.

In the service system described above, an application using a measurement value of the body composition meter may function on the terminal device, and the application may have a function to send the address information of the server to the body composition meter.

A measurement value transmission method of an aspect of the disclosure has: a first step of communication means provided on a body composition meter receiving, from a terminal device capable of communicating with the body composition meter, address information of a server to which a measurement value of the body composition meter is to be sent; a second step of the body composition meter registering the address information received by the communication means; and a third step of sending the measurement value to the server specified by the address information registered by the body composition meter.

A measurement value transmission program of an aspect of the disclosure causes a computer, which is provided on a body composition meter having communication means for receiving, from a terminal device, address information of a server to which a measurement value of the body composition meter is to be sent, to function as: registering means for registering the address information received by the communication means; and transmission control means for sending the measurement value to the server specified by the address information registered by the registering means.

### Advantage of the invention

The present disclosure allows a body composition meter to send a measurement value directly to a server of a web service that a user uses.

### BRIEF DESCRIPTION OF THE DRAWINGS

Figure 1 is a schematic configuration diagram of a service system of an embodiment;
Figure 2 is a function block diagram regarding a function to register address information and a function to send a measurement value of a body composition meter of the embodiment;
Figure 3 is a schematic diagram showing server registration information of the embodiment;
Figure 4 is a flowchart showing the flow of a server address registration process of the embodiment;
Figure 5 is a flowchart showing the flow of a measurement value transmission process of the embodiment; and
Figure 6 is a schematic configuration diagram of a service system of another embodiment.

### MODES OF EMBODYING THE INVENTION

An embodiment of the disclosure will now be described with reference to the drawings. The embodiment described below is merely illustrative of ways to implement the disclosure, and does not limit the disclosure to the specific configurations described below. When the disclosure is to be implemented, any specific configuration may be appropriately adopted according to the mode of implementation.

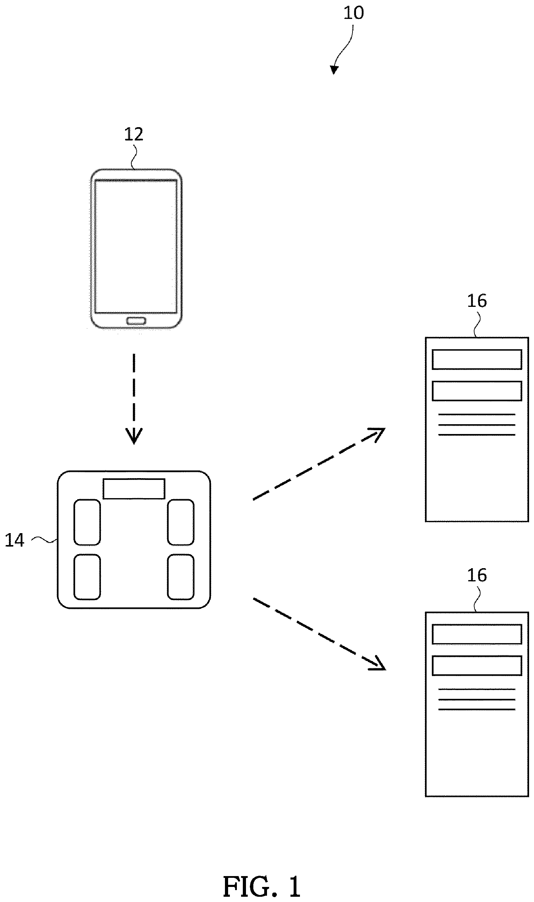

Figure 1 is a schematic configuration diagram of a service system 10 of the embodiment. The service system 10 comprises a terminal device 12, a body composition meter 14, and one or more servers 16.

The terminal device 12 is an information processing apparatus capable of communicating with the body composition meter 14, such as a smartphone, a tablet terminal device, and a laptop computer. The terminal device 12 of the embodiment causes an application using measurement values of the body composition meter 14 to function. This application is, for example, a dedicated application that uses a user's measurement values acquired by the body composition meter 14 to provide a web service to the user (hereinafter referred to as the "service-dedicated app").

Web services include, for example, a weight management service that counsels a user on the user's weight management on the basis of measurement values, and a fitness service that suggests exercises to a user on the basis of measurement values. Weight management and fitness services are provided by different operators, and a user uses a web service appropriate to the user.

The body composition meter 14, which is a biological information measuring device, measures a user's weight and bioimpedance and determines biological information such as a body composition value based on the weight and bioimpedance. The body composition meter 14 calculates, as a body composition value, fat percentage, fat mass, fat-free mass, muscle mass, visceral fat mass, visceral fat level, visceral fat area, subcutaneous fat mass, basal metabolic expenditure, bone mass, body water percentage, BMI, intracellular fluid volume, extracellular fluid volume, or the like. In the following description, the value of weight and biological information measured by the body composition meter 14 is referred to as a measurement value.

The body composition meter 14 of the embodiment also receives user information such as a user's age, sex, and height from the terminal device 12 and holds it. Note that the user information may be directly input to the body composition meter 14. The body composition meter 14 of the embodiment is intended to be used at home by one or more users.

A server 16 receives each user's measurement value from the body composition meter 14 and holds it. Upon request from the service-dedicated app running on the terminal device 12, the server 16 sends the measurement value to the terminal device 12 and causes a display of the terminal device 12 to display the measurement value.

Now, the body composition meter 14 of the embodiment sends a measurement value to a server 16. For this purpose, the body composition meter 14 receives, from the terminal device 12, address information of the server 16 to which the measurement value is to be sent, and registers the address information. Address information of each server 16 is determined for each web service.

For this purpose, the service-dedicated app of the embodiment has a function to send address information of a server 16 to the body composition meter 14 (hereinafter referred to as the "address information transmission function"). A user uses the address information transmission function to send address information of a server 16 corresponding to a web service to the body composition meter 14 and causes the address information to be registered there. Note that information to be sent to (registered on) a server 16 includes: a condition for sending a measurement value to the server 16 (hereinafter referred to as a "transmission condition"); user information; a dedicated encryption certificate corresponding to the web service; and the like, besides address information. An encryption certificate is used when a measurement value is encrypted and sent from the body composition meter 14 to a server 16.

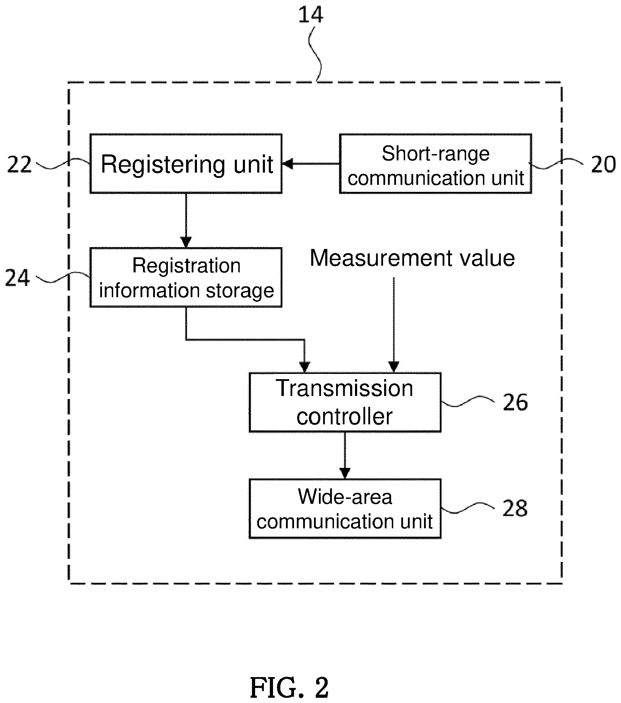

Figure 2 is a function block diagram regarding a function to register address information and a function to send a measurement value of the body composition meter 14 of the embodiment. The body composition meter 14 comprises a short-range communication unit 20, a registering unit 22, a registration information storage 24, a transmission controller 26, and a wide-area communication unit 28. Each function shown in Figure 2 may be, for example, executed by an arithmetic unit of the body composition meter 14 running a program or realized by an individual hardware component such as an ASIC (Application Specific Integrated Circuit).

The short-range communication unit 20 communicates with the terminal device 12 or other information processing apparatuses or the like by a wireless communication standard for narrow area communications. The short-range communication unit 20 of the embodiment communicates with the terminal device 12 via, for example, BLE (Bluetooth Low Energy), but the wireless communications may be performed by other standards. The short-range communication unit 20 receives address information of a server 16 to which a measurement value of the body composition meter 14 is to be sent, a transmission condition, user information, or the like from the terminal device 12.

The registering unit 22 registers address information of a server 16 or the like received by the short-range communication unit 20. Note that the term registering means to cause the address information or the like to be stored in the registration information storage 24.

The registration information storage 24 is, for example, a non-volatile memory such as a flash memory, and holds a variety of user-related information such as address information.

The transmission controller 26 controls the wide-area communication unit 28 to send a measurement value to a server 16 specified by address information registered by the registering unit 22. Note that the transmission controller 26 of the embodiment sends a measurement value to a server 16 if a transmission condition is satisfied.

The wide-area communication unit 28 communicates with a server 16 or other information processing apparatuses by a wireless communication standard for communications whose communication area is wider than the short-range communication unit 20. The wide-area communication unit 28 of the embodiment communicates with a server 16 via, for example, a wireless LAN (Local Area Network) through a router, but the wireless communications may be performed by other standards. The wide-area communication unit 28 sends a measurement value of the body composition meter 14 to a server 16. Note that communications of the body composition meter 14 are not limited to a wireless LAN but may be done through 4G, 5G, or the like with a SIM (Subscriber Identity Module) card equipped on the body composition meter 14.

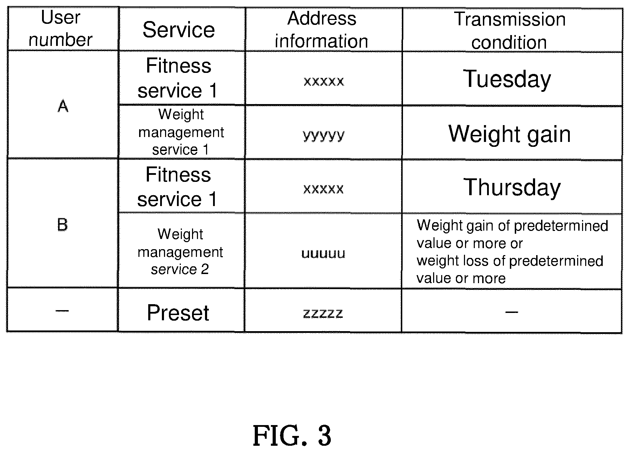

Figure 3 is a schematic diagram showing server registration information stored in the registration information storage 24. Server registration information includes, for example, items of User number, Service, Address information, and Transmission condition. User number is a different number given for each registered user, and comprises, for example, numerals, alphabet letters, or a combination of these. Service is the name of a web service.

The registering unit 22 of the embodiment registers address information for each user. This allows the body composition meter 14 of the embodiment to send a measurement value to a different server 16 for each user. The registering unit 22 of the embodiment also registers a transmission condition for sending a measurement value of the body composition meter 14 to a server 16 in association with address information. The transmission controller 26 sends a measurement value that satisfies a transmission condition to a server 16 specified by address information associated with the transmission condition. This allows the body composition meter 14 of the embodiment to send a measurement value desired by a user to a server 16.

In the example in Figure 3, address information xxxxx and a transmission condition, Tuesday, are registered as server registration information of User A for receiving a service of Fitness service 1. That is, in order for User A to receive the service of Fitness service 1, a measurement value taken on Tuesday is sent to a server 16 specified by the address information xxxxx.

Likewise, in order for User A to receive a service of Weight management service 1, a measurement value is sent to a server 16 specified by address information yyyyy when the weight increases compared to the previous measurement result.

Additionally in the example in Figure 3, in order for User B to receive a service of Fitness service 1, a measurement value taken on Thursday is sent to the server 16 specified by the address information xxxxx. Moreover, in order for User B to receive a service provided by Weight management service 2, a measurement value is sent to a server 16 specified by address information uuuuu when the weight increases by a predetermined value or more compared to the previous measurement result or when the weight decreases by a predetermined value or more compared to the previous measurement result.

As for transmission conditions, a variety of conditions may be registered as well as conditions based on the magnitude of a measurement value and conditions based on a measurement value's acquired date and time or day of week as described above. For example, there may be registered a transmission condition that every measurement value is sent. There may also be a transmission condition that data with a measurement failure is sent to a server other than a server 16. This facilitates the maker's error analysis. Transmission conditions can be changed as appropriate by a user via the service-dedicated app. One transmission condition may be registered for different pieces of address information.

Address information zzzzz for a service named Preset is of a server 16 to which a measurement value is sent when a user's registration information is not stored at all, and is registered in advance in association with no user. There may also be a configuration in which a measurement value is not sent to a server 16 when no registration information is registered. Moreover, a measurement value may be sent to the server specified by the address information zzzzz for Preset regardless of whether address information of a server 16 is registered or not.

A server 16 to which a measurement value is to be sent may be chosen by a user. For this purpose, the registering unit 22 registers a server 16 chosen by a user, as the server 16 to which a measurement value is to be sent. This allows the user to easily change the server 16 to which a measurement value is to be sent. Specifically, when a user wants to change a web service to use or when a user wants to determine a web service on the user's own depending on the progress of weight management, the user chooses a server 16 corresponding to the web service.

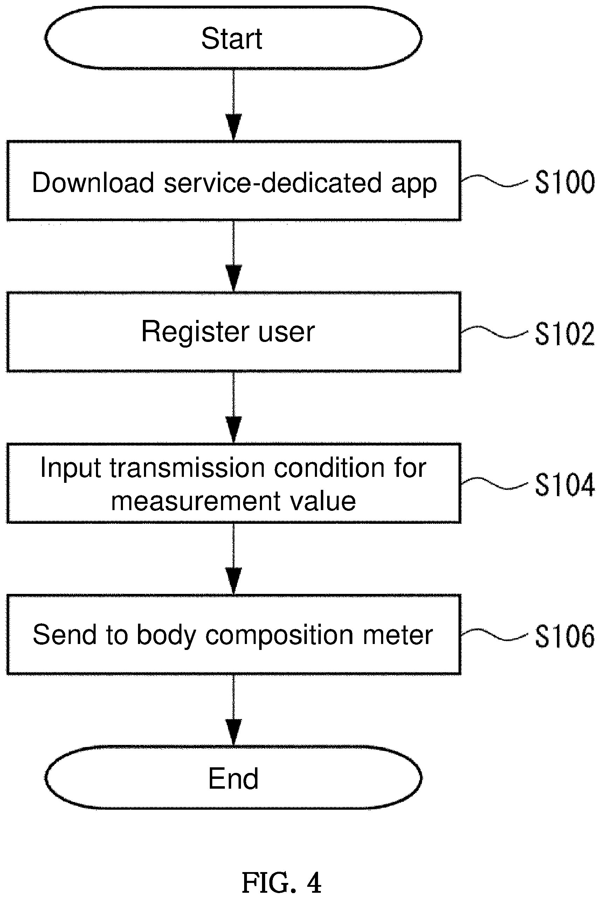

Figure 4 is a flowchart showing the flow of a server address registration process of the embodiment.

First at a step 100, a user downloads and installs the service-dedicated app for the user to receive a desired web service to the user's terminal device 12.

At the next step 102, the user uses the service-dedicated app to register the user for the web service.

At the next step 104, the user uses the service-dedicated app to input a transmission condition for a measurement value of the body composition meter 14.

At the next step 106, the user uses the address information transmission function of the service-dedicated app to send address information, the transmission condition, and the like of a server 16 to the body composition meter 14. Note that the address information is set in the service-dedicated app in advance.

Upon receiving the address information, the transmission condition, and the like, the body composition meter 14 registers them for each user through the registering unit 22 causing them to be stored in the registration information storage 24. Note that the registering unit 22 adds the sent address information, transmission condition, and the like to the server registration information in association with user number registered in the body composition meter 14.

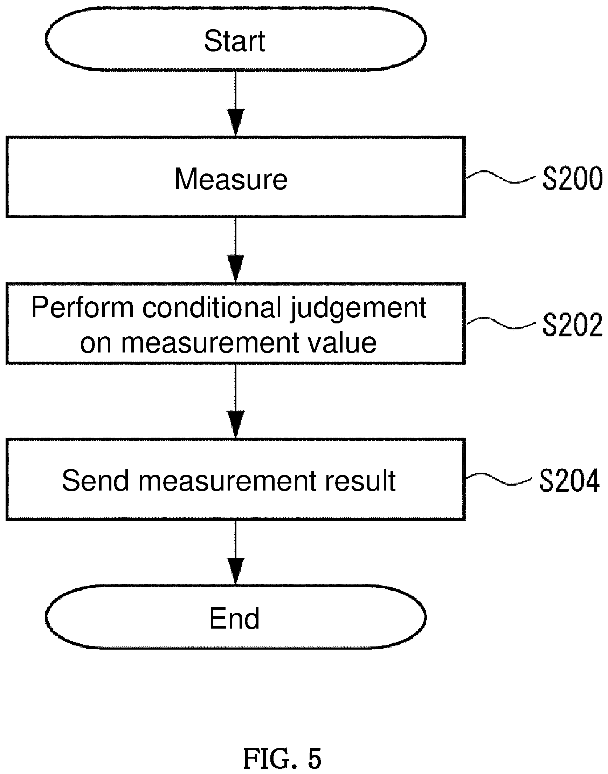

The flow of a measurement value transmission process performed by the body composition meter 14 will be described next with reference to a flowchart in Figure 5. The server address registration process shown in Figure 4 requires to be complete already in order for the measurement value transmission process to be performed.

First at a step S200, a user inputs the user's user number registered in the body composition meter 14 in advance, and measures the user's body composition with the body composition meter 14. The body composition meter 14 thus acquires a measurement value of the user.

At the next step S202, the transmission controller 26 performs a conditional judgement on the measurement value. That is, the transmission controller 26 refers to server registration information stored in the registration information storage 24 and determines a server 16 that satisfies a transmission condition.

At the next step S204, the transmission controller 26 sends the measurement value to the server 16 via the wide-area communication unit 28. The transmission of the measurement value to the server 16 is performed, for example, with encryption using an encryption certificate. Upon receiving the measurement value from the body composition meter 14, the server 16 stores the measurement value in a predetermined storage area in association with the user.

In this way, the body composition meter 14 of the embodiment receives, from the terminal device 12 on which the service-dedicated app for using a measurement value of the body composition meter 14 to provide a service to a user functions, address information of a server 16 to which the measurement value is to be sent. The body composition meter 14 then registers the received address information for each user and sends the measurement value to the server 16 specified by the registered address information.

Consequently, the body composition meter 14 of the embodiment can send a measurement value directly to a server 16 of a web service that a user uses, without a transmission process in which the measurement value is sent to the server 16 via the terminal device 12 or a transmission process in which the measurement value is sent to the server 16 via a predetermined server (inter-server coordination).

While the disclosure has been described with reference to the above embodiment, the technical scope of the disclosure is not limited to the scope provided by the embodiment. Various modifications or improvements can be made to the embodiment without departing from the gist of the disclosure, and those added with the modifications or improvements are also included in the technical scope of the disclosure.

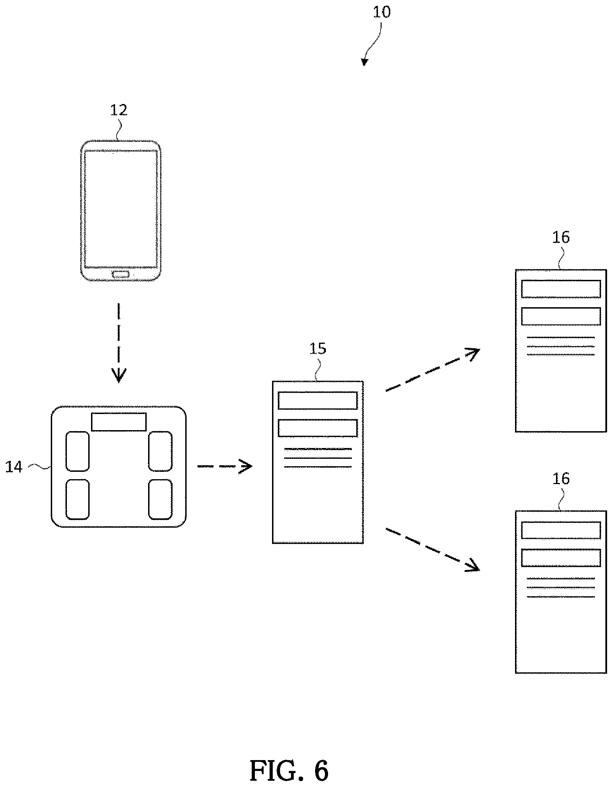

While a description has been made for the above embodiments on a mode in which a measurement value is sent from the body composition meter 14 to a server 16 corresponding to a web service, the disclosure is not limited to this. For example, as shown in Figure 6, there may be a mode in which a measurement value is sent to a server 16 corresponding to a web service via a server 15 that is not related to the web service. The server 15 is, for example, intended to gather measurement values.

In the mode shown in Figure 6, a measurement value of the body composition meter 14 is sent from the body composition meter 14 to the server 15 and is stored in the server 15. The measurement value is then sent from the server 15 to a server 16 indicated by address information, and is also stored in the server 16.

As a specific example, a measurement value is sent from the body composition meter 14 via the server 15 to a server 16 corresponding to a transmission condition or a server 16 chosen by a user. The server 15 sends the measurement value to a server 16 indicated by address information appended to the measurement value sent from the body composition meter 14. In this example, the server 15 does not need to hold address information since the body composition meter 14 holds address information. As another example, the server 15 may hold address information and determine a server 16 to which a measurement value is to be sent according to a transmission condition or a user's choice.

While a description has also been made for the above embodiments on a mode in which a measurement value taken by the body composition meter 14 is sent to a server 16, the disclosure is not limited to this. A measurement value taken not only by the body composition meter 14 but also by an active mass meter, a thermometer, a sphygmomanometer, a scale, a sleep monitor, or other biological information measuring devices may be sent to a server 16.

Note that a transmission condition may depend on the kind of the biological information measuring device. For example, if the biological information measuring device is a sphygmomanometer, a measurement value is sent to a server 16 that provides a medical service when the measurement value is equal to or greater than a reference value. For example, if the biological information measuring device is a sleep monitor, a measurement value is sent to a predetermined server 16 intended for recording when there is a symptom of apnea syndrome. There may also be a case in which when a sleep monitor detects a user's getting out of bed, the fact that the user got out of bed is sent to a predetermined server 16 intended for notification.

While a description has also been made for the above embodiments on a mode in which a web service that a user uses is provided through the service-dedicated app installed on the terminal device 12, the disclosure is not limited to this. A web service provided to a user may be provided via, for example, a website displayed on a browser. In this mode, the application installed on the terminal device 12 to function is a web browser.

A server 16 to which a measurement value is to be sent from the body composition meter 14 is not limited to a server of a web service and may be, for example, a server or the like that configures the settings for the body composition meter 14.

A measurement value acquired by the body composition meter 14 may be sent to the terminal device 12 of a user via the short-range communication unit 20 as well as to a server 16.

### DESCRIPTION OF THE SYMBOLS

- 10:: Service system
- 12:: Terminal device
- 14:: Body composition meter
- 16:: Server
- 20:: Short-range communication unit (communication means)
- 22:: Registering unit (registering means)
- 26:: Transmission controller (transmission control means)

## Claims

1. A body composition meter comprising:
communication means for receiving, from a terminal device capable of communicating with the body composition meter, address information of a server to which a measurement value of the body composition meter is to be sent;
registering means for registering the address information received by the communication means; and
transmission control means for sending the measurement value to the server specified by the address information registered by the registering means.

2. The body composition meter according to claim 1, wherein the registering means registers the address information for each user.

3. The body composition meter according to claim 1 or 2,
wherein the registering means registers a condition for sending the measurement value to the server in association with the address information, and
wherein the transmission control means sends the measurement value that satisfies the condition to the server specified by the address information associated with the condition.

4. The body composition meter according to claim 3, wherein the condition is based on the measurement value's magnitude.

5. The body composition meter according to claim 3 or 4, wherein the condition is based on the measurement value's acquired date and time or day of week.

6. The body composition meter according to any one of claims 1 to 5, wherein the registering means registers a server chosen by a user, as the server to which the measurement value is to be sent.

7. A service system comprising:
a terminal device capable of communicating with a body composition meter;
a body composition meter according to any one of claims 1 to 6; and
a server for holding a measurement value of the body composition meter.

8. The service system according to claim 7,
wherein an application using a measurement value of the body composition meter functions on the terminal device, and
wherein the application has a function to send the address information of the server to the body composition meter.

9. A measurement value transmission method having:
a first step of communication means provided on a body composition meter receiving, from a terminal device capable of communicating with the body composition meter, address information of a server to which a measurement value of the body composition meter is to be sent;
a second step of the body composition meter registering the address information received by the communication means; and
a third step of sending the measurement value to the server specified by the address information registered by the body composition meter.

10. A measurement value transmission program causing a computer, which is provided on a body composition meter having communication means for receiving, from a terminal device, address information of a server to which a measurement value of the body composition meter is to be sent, to function as:
registering means for registering the address information received by the communication means; and
transmission control means for sending the measurement value to the server specified by the address information registered by the registering means.

11. A computer-readable non-transitory storage medium holding the measurement value transmission program according to claim 10.
